(19) Europäisches Patentamt
European Patent Office
Office européen des brevets

(11) EP 0 612 216 B1

# (12) EUROPEAN PATENT SPECIFICATION

(45) Date of publication and mention of the grant of the patent:
**27.09.2000 Bulletin 2000/39**

(21) Application number: **92923557.0**

(22) Date of filing: **28.10.1992**

(51) Int. Cl.$^7$: **A01N 57/12**, A61K 31/685, C07F 9/09

(86) International application number:
**PCT/US92/09179**

(87) International publication number:
**WO 93/08807 (13.05.1993 Gazette 1993/12)**

## (54) PHOSPHOLIPID ANTIMICROBIAL COMPOSITIONS

PHOSPHOLIPID-ANTIMIKROBIELLE ZUSAMMENSETZUNGEN

COMPOSITIONS PHOSPHOLIPIDES ANTIMICROBIENNES

(84) Designated Contracting States:
**AT BE CH DE ES FR GB IT LI NL**

(30) Priority: **28.10.1991 US 784154**
**19.06.1992 US 901204**
**19.06.1992 US 901205**

(43) Date of publication of application:
**31.08.1994 Bulletin 1994/35**

(73) Proprietors:
- **FOST, Dennis L.**
  **Ridgewood, NJ 07450 (US)**
- **PERELLA, James E.**
  **Upper Saddle River, NJ 07458 (US)**
- **KOMOR, Joseph A.**
  **Ramsey, NJ 07446 (US)**

(72) Inventors:
- **FOST, Dennis L.**
  **Ridgewood, NJ 07450 (US)**
- **PERELLA, James E.**
  **Upper Saddle River, NJ 07458 (US)**
- **KOMOR, Joseph A.**
  **Ramsey, NJ 07446 (US)**

(74) Representative:
**Schmitz, Jean-Marie et al**
**Dennemeyer & Associates Sàrl**
**P.O. Box 1502**
**1015 Luxembourg (LU)**

(56) References cited:
**FR-A- 2 461 715**
**US-A- 3 304 349**
**US-A- 4 503 002**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7 (HRS)/3.6

## Description

The present invention concerns antimicrobial agents, a method of inhibiting the growth of microorganisms, personal care and household cleaning compositions, a method of preparing antimicrobial agents, a method of providing protection to a substrate and a method for providing spermicidal and virucidal protection to a substrate.

The present invention relate, to novel antimicrobial compositions and, more particularly, to a class of compounds having specific quaternized amine compounds linked to specific phosphate eaters which exhibit broad spectrum bactericidal and fungicidal activity as well as spermicidal and virucidal activity referred to hereinafter as "antimicrobial phospholipids". The phospholipid compositions of the invention are well tolerated by human tissue making them suitable for use as preservative and disinfectant components in the preparation of personal care, household cleaning germicidal disinfectant and cleaning and like products which exhibit enhanced antimicrobial, antifungal and virucidal characteristics, and in the preparation of therapeutic, personal care and the like products useful as a contraceptive and for the immobilization and/or killing of human and animal sperm.

Phosphate ester and quaternary amine compounds are well known and have been widely used for many years for a variety of applications including those requiring surfactant properties. Known phosphate esters do not generally exhibit any antimicrobial characteristics, and while quaternary amine compounds are known in general to exhibit antimicrobial activity, such compounds are extremely irritating and thus have limited usefulness in personal care and cosmetic products. More recently, various betaine-type derivatives having, in general, quaternized alkyl amine groups and at least one phosphorous-containing anion in the molecule referred to hereinafter as "synthetic phospholipids", have been disclosed and suggested as, for example, in US-A-4,215,064, 4,233,192 and 4,380,637 to Lindemann et at., US-A-4,209,449, 4,336,385 and 4,503,002 to Mayhew et al., and US-A-4,243,602, 4,283,542 and 4,336,386 to O'Lenick et al. These synthetic phospholipids are suggested as exhibiting an outstanding combination of surfactant characteristics as well as being well tolerated by human tissue, i.e., they exhibit exceptionally low ocular irritation and oral toxicity. While these known phospholipids have been found useful as surfactants in a variety of personal care, household cleaning and the like products, such products also require the incorporation of antimicrobial preservatives to inhibit microbial spoilage and increase shelf life, and there is no suggestion that any of these compounds exhibit spermicidal and/or virucidal activity.

The US-A-4,503,002 describes phosphate quaternary compounds of the formula

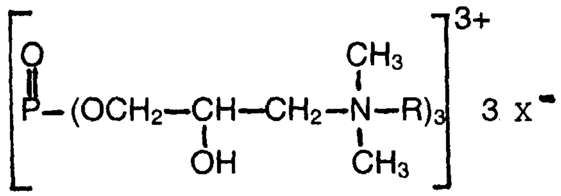

wherein R is a tertiary amine group of from 6 to 40 carbon atoms, and X is an anion. These compounds have bacteriostatic properties.

The FR-A-24 61 715 describes cationic triesters of phosphoric acid having bacteriostatic properties.

US-A-3304349 discloses mono-esters of quaternary ammonium compounds with phosphate, useful as antimicrobial agents such as spermicides or virucides.

It is well known that there is a need for effective preservatives in a wide variety of applications where inhibiting the growth of microorganisms is necessary, as for example, personal care products such as shampoos, creams, lotions, cosmetics, liquid soaps, and household products such as fabric cleaners and softeners, hard surface cleaners and the like. The shelf life of these preparations depends on their resistance to microbial spoilage. In addition, antimicrobial agents are a matter of substantial commercial importance in many industrial applications and products such as in paint, wood, textiles, adhesives and sealants, leather, plastics, oil, rubber and metal working fluids etc.

Certain compounds have long been known and used commercially as preservatives. For example, 1,3-dimethylol-5,5-dimethylhydantoin (DMDMH) is useful as a formaldehyde donor for the preservation of personal care products, cosmetics and household products and halopropynyl carbamates are known for their fungicidal activity. Other commercially known preservatives include Quaternium-15 (DOWICIL 200 from Dow Chemical Company); Imidazolidinyl urea (GERMALL 115 from Sutton Laboratories); formaldehyde in the free state, as in formalin; alkyl parabens (e.g. methyl, ethyl and propyl) etc. While such materials have achieved commercial acceptance for many personal care and household products, they generally present a variety of limitations for such use including being unduly expensive; exhibiting limited antimicrobial or antifungal activity, or limited solubility in water; exhibiting undue pH dependence, adverse toxicological properties and skin sensitization or possible carcinogenicity; or they may be inactivated by commonly used materials.

Various synergistic combinations of ingredients have been also suggested for use as preservatives in certain applications such as, for example, disclosed in US-A-3,699,231, 3,929,561, 4,454,146, 4,655,815, but these compositions generally exhibit unfavorable toxicity characteristics, particularly skin and eye irritation, and are not suitable for personal care and household products, and the development of effective, inexpensive, multifunctional products having a broad spectrum activity has long been sought.

In accordance with the present invention there has now been discovered novel antimicrobial agents which surprisingly exhibit both excellent broad spectrum antibacterial and antifungal activity suitable for use as preservative and/or disinfectant agents in a variety of personal care compositions, household cleaning formulations and the like. These agents have also been found to possess potent spermicidal and virucidal activity making them particularly useful as a contraceptive, and for immobilizing and/or killing human and animal sperm for extended periods of time and a variety of infectious viruses. The antimicrobial agents of the present invention are described in claim 1. The method of inhibiting the growth of microorganisms according to the present invention is described in claim 5. The personal care and household cleaning compositions of the present invention are described in claim 6. The method of preparing the antimicrobial agents of the present invention is described in claim 8. The method of providing protection to a substrate is described in claim 11 and the method of providing spermicidal and virucidal protection to a substrate is described in claim 12. The novel antimicrobial agents of the invention comprise particular synthetic phospholipid compounds that may be represented by the following general formula:

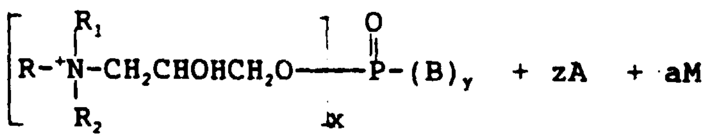

wherein:

x = mixtures of 1 to 3;
x+y = 3 ;
z = x ;
a = 0 to 2;
B = $O^-$ or OM;
A = an anion;
M is a cation;
R, $R_1$ and $R_2$ are the same or different and are alkyl, substituted alkyl, alkyl aryl or alkenyl groups of up to 16 carbon atoms with the proviso that the total carbon atoms in R + $R_1$ + $R_2$ is between 10 and 24.

The particular synthetic antimicrobial phospholipids of the invention not only surprisingly and unexpectedly exhibit broad spectrum bactericidal and fungicidal activity suitable for use as preservative and/or disinfectant agents in personal care and household products, but such phospholipids surprisingly also exhibit potent spermicidal and virucidal activity making them useful, for example, as a contraceptive and in topical and therapeutic compositions for killing and/or immobilization of human and animal sperm and as a disinfectant in hospitals and the like. Even small amounts of the phospholipid compositions of the invention exhibit effective antimicrobial, spermicidal and virucidal activity and the antimicrobial phospholipid compounds of the invention are extremely well tolerated by human tissue, i.e., they exhibit exceptionally low ocular and skin irritation and oral toxicity. Moreover, such agents are substantive to human and animal tissue as well as many known substrate materials such as used in contraceptives and the like and can be used in product formulations containing nonionic, anionic, amphoteric and/or cationic components without significant inhibition or reduction of the required antimicrobial, spermicidal and virucidal activity. The antimicrobial agents of the invention may also be used in combination with other known antimicrobial agents, when desired for particular applications, to enhance the antimicrobial and virucidal efficacy thereof.

In another aspect of the invention, there is provided a method of inhibiting the growth of microorganisms in personal care, household cleaning and the like products which comprises incorporating in a personal care or household cleaning formulation an antimicrobial effective amount of an antimicrobial phospholipid compound of the general formula:

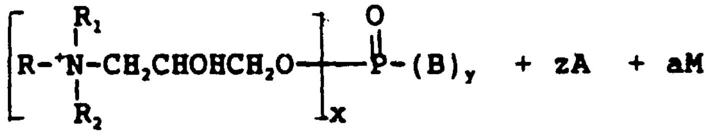

wherein:

x = mixtures of 1 to 3;
x+y = 3 ;
z = x ;
a = 0 to 2;
B = $O^-$ or OM;
A = an anion;
M is a cation;
R, $R_1$ and $R_2$ are the same or different and are alkyl, substituted alkyl, alkyl aryl or alkenyl groups of up to 16 carbon atoms with the proviso that the total carbon atoms in R + $R_1$ + $R_2$ is between 10 and 24.

In a further aspect of the present invention, there is provided a personal care composition or a household cleaning composition which comprises a surface active agent and an antimicrobial effective amount of an antimicrobial phospholipid compound component of the general formula:

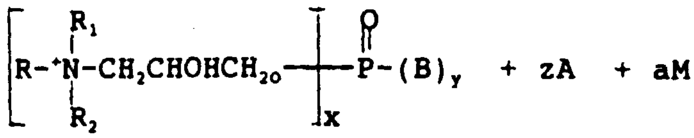

wherein:

x = mixtures of 1 to 3;
x+y = 3 ;
z = x ;
a = 0 to 2;
B = $O^-$ or OM;
A = an anion;
M is a cation;
R, $R_1$ and $R_2$ are the same or different and are alkyl, substituted alkyl, alkyl aryl or alkenyl groups of up to 16 carbon atoms with the proviso that the total carbon atoms in R + $R_1$ + $R_2$ is between 10 and 24.

In a still further aspect of the invention there is provided a method of preparing an antimicrobial compound which exhibits broad spectrum antibacterial and antifungal activity suitable for use as an antimicrobial agent in personal care and household products, said antimicrobial compound comprising an antimicrobial phospholipid that may be represented by the general formula:

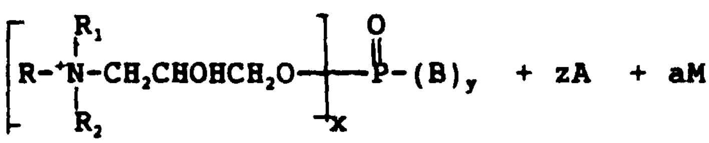

wherein:

x = mixtures of 1 to 3;

x+y = 3 ;
z = x ;
a = 0 to 2;
B = $O^-$ or OM;
A = an anion;
M is a cation;
R, $R_1$ and $R_2$ are the same or different and are alkyl, substituted alkyl, alkyl aryl or alkenyl groups of up to 16 carbon atoms with the proviso that the total carbon atoms in R + $R_1$ + $R_2$ is between 10 and 24.

which comprises:

reacting a phosphate ester reactant with a tertiary amine in the molar ratio of from 1:1 to 3:1, and preferably from about 2.0:1 to 2.5:1, of amine to phosphate ester until the tertiary amine is completely reacted, said phosphate ester reactant being of the general formula:

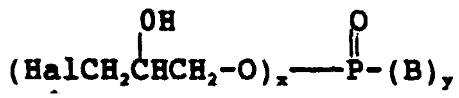

wherein:

x = mixtures of 1 to 3
x+y = 3
B = $O^-$ or OM
Hal = halogen; and

said tertiary amine being of the general formula:

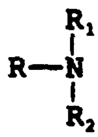

wherein R, $R_1$ and $R_2$ is the same or different and are alkyl, substituted alkyl, alkyl aryl or alkenyl groups of up to 16 carbon atoms with the proviso that the total carbon atoms in R +$R_1$ + $R_2$ is between 10 and 24.

In yet another aspect of the invention there are provided compositions for topical or therapeutic use in the killing and/or immobilizing of human and animal sperm including contraceptive protection which comprises a spermicidally effective amount of a antimicrobial phospholipid agent of the general formula:

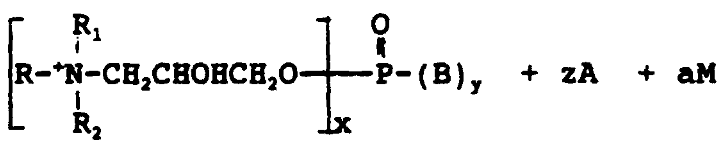

wherein:

x = mixtures of 1 to 3
x+y = 3 ;
z = x ;
a = 0 to 2;
B = $O^-$ or OM;
A = an anion;
M is a cation;

R, $R_1$ and $R_2$ are the same or different and are alkyl, substituted alkyl, alkyl aryl or alkenyl groups of up to 16 carbon atoms with the proviso that the total carbon atoms in R + $R_1$ + $R_2$ is between 10 and 24;

a spermicidal agent of the general formula:

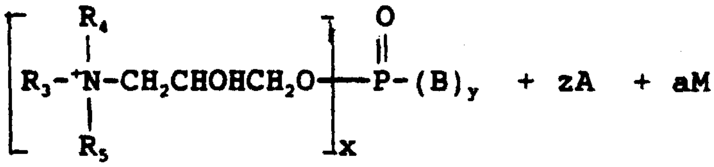

wherein:

x is as hereinabove defined;
x+y = 3 ;
z = x ;
a = 0 to 2;
B = $0^-$ or OM;
A is on Anion;
M is a Cation;
$R_3$ is an amidoamine moiety of the formula:

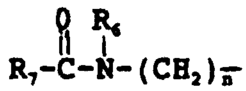

wherein:

$R_7$ is alkyl, alkenyl, alkoxy or hydroxyalkyl of from 5 to 21 carbon atoms each, or aryl or alkaryl of up to 20 carbon atoms;
$R_6$ is hydrogen or alkyl, hydroxyalkyl or alkenyl of up to 6 carbon atoms each or cycloalkyl of up to 6 carbon atoms, preferably of from 2 to 5 carbon atoms, or polyoxyalkylene of up to 10 carbon atoms; and
n is an integer from 2 to 6; and

$R_4$ and $R_5$, which may be the same or different, are selected from alkyl, hydroxyalkyl, carboxyalkyl of up to 6 carbon atoms in each alkyl moiety, and polyoxyalkylene of up to 10 carbon atoms; in addition
$R_4$ and $R_5$ taken together with the nitrogen to which they are attached may represent an N-heterocycle;

or mixture thereof.

In still another aspect of the invention there are provided compositions for use in the killing and/or immobilizing a variety of infectious viral organisms including disinfectant protection which comprise a virucidally effective amount of a antimicrobial phospholipid agent of the general formula:

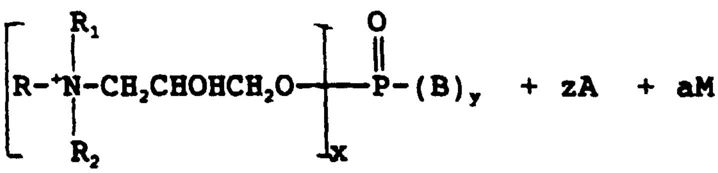

wherein:

x = mixtures of 1 to 3
x+y = 3 ;

z = x ;
a = 0 to 2;
B = $O^-$ or OM;
A = an anion;
M is a cation;
R, $R_1$ and $R_2$ are the same or different and are alkyl, substituted alkyl, alkyl aryl or alkenyl groups of up to 16 carbon atoms with the proviso that the total carbon atoms in R + $R_1$ + $R_2$ is between 10 and 24;

a virucidal agent of the general formula:

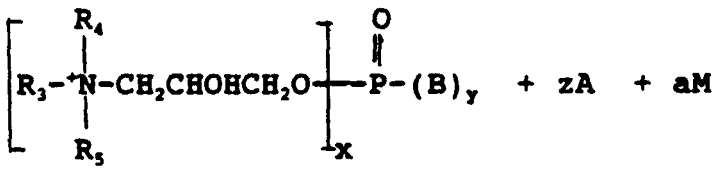

wherein:

x is as hereinabove defined;
x+y = 3 ;
z = x ;
a = 0 to 2;
B = $0^-$ or OM;
A is on Anion;
M is a Cation;
$R_3$ is an amidoamine moiety of the formula:

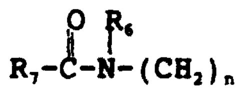

wherein:

$R_7$ is alkyl, alkenyl, alkoxy or hydroxyalkyl of from 5 to 21 carbon atoms each, or aryl or alkaryl of up to 20 carbon atoms;
$R_6$ is hydrogen or alkyl, hydroxyalkyl or alkenyl of up to 6 carbon atoms each or cycloalkyl of up to 6 carbon atoms, preferably of from 2 to 5 carbon atoms, or polyoxyalkylene of up to 10 carbon atoms; and
n is an integer from 2 to 6; and

$R_4$ and $R_5$, which may be the same or different, are selected from alkyl, hydroxyalkyl, carboxyalkyl of up to 6 carbon atoms in each alkyl moiety, and polyoxyalkylene of up to 10 carbon atoms;
in addition $R_4$ and $R_5$ taken together with the nitrogen to which they are attached may represent an N-heterocycle;

or mixtures thereof.

As used herein the phrases "antimicrobial" and "inhibiting microbial growth" describes the killing of, as well as the inhibition or control of the growth of bacteria (gram positive and gram negative), fungi, yeasts and molds.

As used herein the phrase "spermicidal" describes sperm immobilization as well as the killing of human and animal sperm.

As used herein the phrase "virucidal" describes the killing of as well as the immobilization of infectious virus organisms.

The present invention is directed to novel antimicrobial agents which surprisingly and unexpectedly exhibit excellent broad spectrum bactericidal and fungicidal activity and effectiveness and effectively inhibit the growth of a variety of bacteria, yeasts and molds, as well as possessing potent spermicidal and virucidal killing and/or immobilizing activity for human and animal sperm and a variety of infectious viruses. Moreover, such active agents may be used in combination with or in the presence of anionic, nonionic, amphoteric and/or cationic surfactants without inhibition of the antimicrobial, spermicidal and virucidal efficacy thereof and are virtually non-irritating to the skin and eyes; thus, such

antimicrobial agents may be used in diverse formulations and applications.

The novel antimicrobial agents of the present invention comprise a class of synthetic "antimicrobial phospholipid" compounds which may be represented by the following general formula:

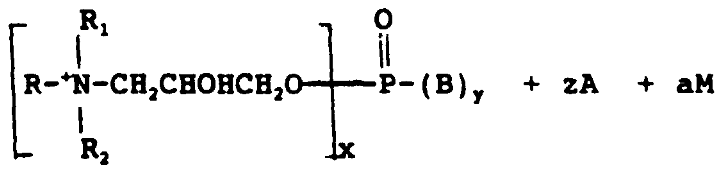

wherein:

x = mixtures of 1 to 3;
x+y = 3 ;
z = x ;
a = 0 to 2;
B = $O^-$ or OM;
A = an anion;
M is a cation;
R, $R_1$ and $R_2$ are the same or different and are alkyl, substituted alkyl, alkyl aryl or alkenyl groups of up to 16 carbon atoms with the proviso that the total carbon atoms in R + $R_1$ + $R_2$ is between 10 an 24;

The antimicrobial phospholipid compounds described which, as indicated, exhibit broad spectrum antimicrobial activity as well as being substantially non-irritating to humans can be prepared by reaction of tertiary amines and phosphate esters corresponding to the amine and phosphate ester moieties in the above formula. Such compounds can be prepared by reacting the corresponding tertiary amine and phosphate ester reactants in the molar ratio of 1:1 to 3:1, and preferably from 2.0:1 to 2.5:1 of amine to phosphate ester, for the time necessary for the amine to be completely reacted.

Tertiary amines suitable for use in accordance with the practice of the invention can be represented by the general formula:

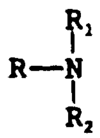

wherein:

R, $R_1$ and $R_2$ is the same or different and are alkyl, substituted alkyl, alkyl aryl, or alkenyl groups of up to 16 carbon atoms with the proviso that the total carbon atoms in R + $R_1$ + $R_2$ is between 10 and 24.

Exemplary tertiary amines include:

tributylamine
(di(hydroxyethyl)hexyl)-amine
bis(2-hydroxyethyl)cocoamine
N,N-dimethyl-dodecylamine
N,N-dimethyl-tetradecylamine
N,N-dimethyl-hexadecylamine
N,N-dimethyl-cocoamine
N,N-dimethyl-cetylamine
dimethyl ($C_8$-$C_{16}$) alkyl amine

The phosphate ester reactants suitable for use in accordance with the practice of the invention can be represented by the general formula:

$$(HalCH_2CHOHCH_2-O)_x - \overset{\overset{\displaystyle O}{\|}}{P} - (B)_y$$

wherein:

x = mixtures of 1 to 3
x + y = 3
B = O - or OM
Hal - halogen

The phosphate ester intermediate may be prepared by known procedures wherein phosphoric acid and various phosphate salts, and preferably monosodium phosphate, are reacted in an aqueous medium with epichlorohydrin, generally in the molar ratio of about 1:3, until the reaction is complete.

As noted, the instant invention is based upon the discovery that the antimicrobial phospholipid compounds of the invention described above are effective in controlling the growth of bacteria, yeasts and molds in diverse formulations and applications such as cosmetic, toiletries, personal care, household and related products and materials. The antimicrobial agents of the invention are not only an effective antimicrobial for the destruction or control of fungi and bacteria that cause degradation and deterioration of diverse personal care and household product formulations, but also by their activity against the organisms that can reside and accumulate on various surfaces, they can provide utility in sanitizing, disinfecting and bacteriostatic applications.

The antimicrobial activity of the compounds described above has been confirmed using standard laboratory techniques, including the Minimum Inhibitory Concentration (MIC) technique. They have been found effective, for example, in inhibiting bacteria including S. aureus, E. coli, P. aeruginosa and S. choleraesuis. They have also been found effective against yeast and mold including C. albicans and A. niger. In these tests it has been determined that the presence of anionic, nonionic, amphoteric and/or cationic materials did not inhibit the antimicrobial efficacy nor did a variety of inactivators commonly encountered in personal care and household applications. The broad spectrum preservative characteristics of the antimicrobial phospholipids of the invention in typical cosmetic formulations have also been established and confirmed.

Specifically, molds and yeasts which may be inhibited include Aspergillus niger, Candida albicans plus various species of Penicillium, Tricholphyton, Alternaria, Gliocladium, Paecilomyces, Mucor, Fusarium, Geotrichum, Cladosporium and Trichoderma. Examples of the bacteria include Salmonella choleraesuis, Serratia marcescens, Klebsiella pneumoniae, Enterobacter aerogenes, Aerobacter aerogenes, Proteus vulgaris, Streptococcus faecalis, Pseudomonas aeruginosa, Escherichia coli, Staphylococcus aureus, Staphylococcus epidermidis, M. luteus, P. mirabilis, P. cepacia, P. stutzeri and A. hydrophilia.

Another aspect of the present invention is the discovery that the antimicrobial phospholipid compounds surprisingly and unexpectedly exhibit significant spermicidal and antiviral activity which further enhances the utility of the compounds of the invention for a diversity of applications.

The spermicidal activity of the phospholipid compounds described above has been confirmed using test methodology based on the International Planned Parenthood Federation (IPPF) spermicidal assay as set forth in 21CFR, Part 351, Volume 45, No. 241. Substantivity to human skin as well as known latex and fabric substrate materials treated with aqueous solutions of the phospholipid compounds that were submitted to "repeat washing microbiological test protocol" have shown such compounds to possess residual antimicrobial activity for extended periods of time.

The virucidal activity of the phospholipid compounds described above has been confirmed using test methodology according to U.S. Environmental Protection Agency guidelines for determining the virucidal efficacy of disinfectants intended for use on dry inanimate environmental surfaces (U.S. E.P.A. Pesticide Assessment Guideline, subdivision G, Product Performance, 198, Section 91-30 pp 72-76).

Specifically, virucidal efficacy has been found against Human Influenza A virus; Herpes Simplex, type 2, virus; and the Human Immunodeficiency Virus (HIV).

The antimicrobial phospholipid compounds described above have activity against bacteria, yeasts and molds as well as human and animal sperm and a variety of infectious viral organism when employed at appropriate levels of concentration and may be used to inhibit growth or effectively destroy these organisms. It should be obvious that the required effective concentration or amount will vary with particular organisms and also on a number of other factors in particular applications. In general, however, effective antimicrobial response is obtained when the active agent is employed in concentrations ranging between five and 10,000 ppm (parts per million) and preferably between about 50 and 1,000 ppm. Generally, the concentration of the agent required for bactericidal activity will be lower than the concentration required for fungicidal activity, and the concentration of the agent required for spermicidal and virucidal activity will generally be the same or higher than the concentration required for fungicidal activity.

For other applications, amounts of from 0.04% to about 5% or higher, and preferably 0.07% to 3.0%, by weight of the active agent of the present invention is incorporated into a composition or sprayed onto or otherwise applied to a substrate to be treated in order to prevent growth of bacteria, yeasts and molds as well as killing human and animal sperm and infectious viral organisms. It will also be understood that the antimicrobial agents of the invention may be used in combination with other antimicrobial, spermicidal and/or virucidal materials.

The compatibility of the antimicrobial phospholipid compounds of the invention with human tissue, i.e., dermal and eye tissue has also been tested. In these tests, 48 hour human patch dermal evaluations (5% in water), in vitro ocular evaluations (3% in water) and repeated insult patch tests (3% in water) determined that the compounds are substantially non-irritating to humans, they are safe and suitable for use in eye area products and are not a skin sensitizer to humans.

While the phospholipid compounds hereinabove described exhibit broad spectrum antimicrobial as well as potent spermicidal and virucidal activity, certain other phospholipid compounds surprisingly have also been found to possess potent spermicidal and virucidal activity. Such compounds are also compatible with anionic, nonionic, amphoteric and/or cationic materials without inhibition of their spermicidal and virucidal efficacy and exhibit low sensitivity to human tissue.

Phospolipid compounds which are also suitable as spermicidal and virucidal agents have the general formula:

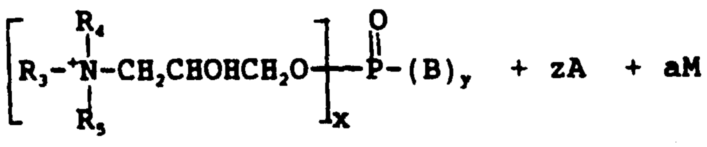

wherein:

x = 1 to 3 or mixtures thereof;
x+y = 3 ;
z = x ;
a = 0 to 2;
B = $0^-$ or OM;
A is on Anion;
M is a Cation;
$R_3$ is an amidoamine moiety of the formula:

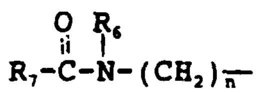

wherein:

$R_7$ is alkyl, alkenyl, alkoxy or hydroxyalkyl of from 5 to 21 carbon atoms each, or aryl or alkaryl of up to 20 carbon atoms;
$R_6$ is hydrogen or alkyl, hydroxyalkyl or alkenyl of up to 6 carbon atoms each or cycloalkyl of up to 6 carbon atoms, preferably of from 2 to 5 carbon atoms, or polyoxyalkylene of up to 10 carbon atoms; and
n is an integer from 2 to 6; and

$R_4$ and $R_5$, which may be the same or different, are selected from alkyl, hydroxyalkyl, carboxyalkyl of up to 6 carbon atoms in each alkyl moiety, and polyoxyalkylene of up to 10 carbon atoms; in addition
$R_4$ and $R_5$ taken together with the nitrogen to which they are attached may represent an N-heterocycle.

The antimicrobial phospholipid compounds of the invention may be incorporated in diverse personal care and household product formulations as, for example, a preservative therefore and/or as a disinfectant agent, and the incorporation of the compounds of the invention into such products can be done in accordance with standard practices.

The active ingredients described can be diluted or otherwise mixed with solvents, dispersants, wetting agents, carriers and the like for topical or therapeutic use as a spermicide or virucide in any desired application formulation such as liquids, sprays, jellies, creams, tablets, suppositories, foams etc. In connection with suitable modes of application for spermicidal or virucidal results, the phospholipid agents can be mixed with one or more pharmaceutically acceptable solid inert carriers.

The invention will now be further illustrated by reference to certain specific examples which are provided herein for purposes of illustration only and are not intended to limit the scope therein.

<u>Example 1</u>

925.6 grams of soft water are charged to a reaction vessel and heat is applied to 50°C. 554.4 grams of dimethyl cocoamine ($C_{12}$ - 66%; $C_{14}$ - 26%; C16 - 8%) are charged into the reaction vessel under good agitation and heat is applied to 90°C. An aqueous solution of 938.8 grams of 40% active 2-propanol, 1 - chlorophosphate (3:1) are charged into the reaction vessel in four equal increments over 1.5 hours using good agitation while maintaining the temperature at 90 - 95°C. Heating is continued at 90 - 95°C. until the pH (10%) is 6.5 or less and the percentage of free tertiary amine is 0.5% maximum; approximately six to nine hours. The reaction mixture is then cooled to 80°C., 55.2 grams of 50% NaOH are charged into the reaction vessel and the reaction mixture is heated back to 90°C. Heating at 90°C. is continued until the percentage of NaCl is 6.9± 0.2 %, approximately one hour. The reaction mixture is then cooled to 50°C. and the pH (10%) is adjusted to 7.0± 0.5 with citric acid (approximately 9.7 grams). 22.1 grams of $H_2O_2$ (35%) are charged to the reaction vessel with good agitation and heat is applied to 90°C. and maintained for one hour. The reaction mixture is then cooled to 50°C. and discharged. The product is a clear liquid having ≤0.5% free amine, a pH (10%) of 7.0± 0.5 and a specific gravity @ 25°C. of 1.05.

<u>Example 2</u>

682.4 grams of propylene glycol and 453.0 grams of water are charged to a reaction vessel and heat is applied to 50°C. 655.2 grams of dimethyl cetylamine are charged into the reaction vessel with good agitation and heat is applied to 90°C. An aqueous solution of 938.8 grams of 40% active 2-propanol, 1 chlorophosphate (3:1) are divided into four equal increments and charged into the reaction vessel over 1.5 hours while maintaining the temperature at 90 - 95°C. Heating is continued at 90 - 95°C until the pH (10%) is 6.5 or less and the free tertiary amine is ≤0.5%, approximately six to nine hours. The reaction mixture is then cooled to 80°C. and 47.3 grams of 50% NaOH is added with good agitation. Heat is applied to 90°C and maintained until the percentage of NaCl is 6.1± 0.2%, approximately one hour. The reaction mixture is then cooled to 50°C. and the pH (10%) is adjusted to 7.0± 0.5 with citric acid, approximately 4.7 grams being added. 25 grams of 35% $H_2O_2$ are charged into the reaction vessel, heat is applied to 90°C. and maintained for one hour. The reaction mixture is then cooled to 50°C. and discharged.

The product is a clear liquid having a specific gravity @ 25°C. of 1.05, a pH (10%) of 7.0± 0.5 and Free amine of ≤0.5%.

<u>Example 3</u>

The products of Example 1 and Example 2 are screened for antimicrobial activity using a modified Minimum Inhibitory Concentration (MIC) testing protocol. The initial screening is conducted using the following test organisms:

<u>S. aureus</u> ATCC #6538
<u>C. albicans</u> ATCC #10259
<u>A. niger</u> ATCC #6275
<u>Penicillium variable</u> ATCC #XXXX

The growth media used are Brain Heart Infusion Broth for bacteria and Sabouroud Broth for yeast and mold.

A series of ten sequential two-fold dilutions of the test material is made in an appropriate growth promoting culture medium for each organism to be tested. A standard number of microorganisms are inoculated into each of the prepared dilutions containing the medium plus the test material. Inoculated tubes are incubated at appropriate temperature for 72 hours.

Visual readings are taken after 24, 48 and 72 hours. The 72-hour incubated tubes are subcultured on agar media to verify inhibition of growth. Data is recorded as positive or negative for growth at each of the dilutions of the test material under evaluation. The minimum lethal concentration is defined as the smallest concentration of antimicrobial agent that, on subculture, either fails to show growth or results in a 99.9% decrease in the initial concentration of inoculum.

Comparative MIC data of the initial screening test is reported in Table I.

Table I

| Test Organism | Example I Sample | Example II Sample |
|---|---|---|
| S. aureus | 20 ppm | 60 ppm |
| C. Albicans | 20 ppm | 80 ppm |
| A. niger | 10 ppm | 30 ppm |
| P. variable | 10 ppm | 80 ppm |

An additional test panel is conducted to evaluate the products of Example 1 and Example 2. The further tests are conducted with Pseudomonas aeruginosa ATCC #15442, E. coli ATCC #8739 and Salmonella choleraesuis ATCC #10708. The MIC test protocol described above is used in conducting the additional test.

Comparative MIC data of the additional screening test is reported in Table II.

Table II

| Test Organism | Example I | Example 2 |
|---|---|---|
| P. aerugenosa | 80 ppm | 80 ppm |
| E. coli | 20 ppm | 160 ppm |
| S. choleraesuis | 20 ppm | 80 ppm |

As can be seen, both the Example 1 and Example 2 products exhibit significant antimicrobial properties.

Example 4

A series of typical personal care products are prepared by standard practices using the following proportion of ingredients:

Product A Shampoo

| | |
|---|---|
| Sodium Lauryl Sulfate | 15.0% by weight |
| Water | 85.0% |
| Antimicrobial Phospholipid (Example 1) | variable |

Compositions are prepared with the following proportions of the product of Example 1.

| Test Sample | Example 1 Product |
|---|---|
| A-1 | 0.00% by weight |
| A-2 | 0.25% by weight |
| A-3 | 0.50% by weight |
| A-4 | 1.0% by weight |

Product B Make-Up Foundation

| | | | |
|---|---|---|---|
| a) | Steareth - 20 | | 1.5% by weight |
| | Pigment | | 15.0% by weight |
| | 0.5% Kelzan AR/1% NaCl | | 76.0% by weight |
| b) | Steareth - 2 | | 2.5% by weight |
| | Isopropyl Myristate | | 2.0% by weight |
| | Hexyl Laureate | | 2.0% by weight |
| | Dow Fluid 200/100 cs | | 1.0% by weight |
| | Antimicrobial Phospholipid | | variable |
| | Pigment: | White | 13.5% by weight |
| | | Red | 0.15% by weight |
| | | Brown | 1.20% by weight |
| | | Yellow | 0.15% by weight |

Compositions are prepared with the following proportions of the product of Example 1.

| Test Sample | Example 1 Product |
|---|---|
| B-1 | 0.00% by weight |
| B-2 | 0.25% by weight |
| B-3 | 0.50% by weight |
| B-4 | 1.0% by weight |

Product C Lotion

| | | |
|---|---|---|
| a) | Steareth - 20 | 2.0% by weight |
| | Water | 87.5% by weight |
| | Product of Example 1 | variable |
| b) | Steareth - 2 | 3.0% by weight |
| | Isopropyl Myristate | 5.0% by weight |
| | Cetearyl Alcohol | 2.5% by weight |

Compositions are prepared with the following proportions of the product of Example 1.

| Test Sample | Example 1 Product | |
|---|---|---|
| C-1 | Product of Example 1 | 0.0% by weight |
| C-2 | Product of Example 1 | 0.1% by weight |
| C-3 | Product of Example 1 | 0.5% by weight |

Example 5

The personal care products of Example 4 are subject to Preservative Challenge Tests as follows:

Aliquots of each test preparation are inoculated with separate representative mixed cultures of bacteria and fungi. Plate counts to determine survivors are performed at 0 time and after 3, 7, 14, 21 and 28 days of incubation. Bacterial samples showing a less than 10 recovery at 14 days are re-inoculated at 21 days. Results are presented as surviving organisms present at each time interval per gram of product tested.

Product A

INOCULUM

a) Mixed bacteria: Pseud. aeruginosa (ATCC 15442); E.coli (ATCC 8739 or 11229); S. aureus (ATCC 6536).
b) Mixed fungi: A. niger (ATCC 9642); P. luteum (ATCC 9644); C. albicans (ATCC 10231).

| TEST SAMPLE | DAYS | BACTERIA | FUNGI | CONTROL |
|---|---|---|---|---|
| A-1 | 0 | 2,100,000 | 740,000 | <10 |
| | 3 | 17,500 | 4,750 | <10 |
| | 7 | 2,100,000 | 740,000 | <10 |
| | 14 | 2,100,000 | 740,000 | <10 |
| | 21* | 2,100,000 | 740,000 | <10 |
| | 28 | 2,100,000 | 740,000 | <10 |
| A-2 | 0 | 2,100,000 | 740,000 | <10 |
| | 3 | 24,200 | 1,900 | <10 |
| | 7 | <10 | <10 | <10 |
| | 14 | <10 | <10 | <10 |
| | 21* | <10 | <10 | <10 |
| | 28 | <10 | <10 | <10 |
| A-3 | 0 | 2,100,000 | 740,000 | <10 |
| | 3 | 16,900 | 9,700 | <10 |
| | 7 | <10 | <10 | <10 |
| | 14 | <10 | <10 | <10 |
| | 21* | <10 | <10 | <10 |
| | 28 | <10 | <10 | <10 |
| A-4 | 0 | 2,100,000 | 740,000 | <10 |
| | 3 | 23,700 | 1,620 | <10 |
| | 7 | <10 | <10 | <10 |
| | 14 | <10 | <10 | <10 |
| | 21* | <10 | <10 | <10 |
| | 28 | <10 | <10 | <10 |
| NOTE: Control is an uninoculated sample for background count. Bacterial and Fungal Counts are as organisms recovered per gram of sample. Test Day is the number of days after inoculation of the test sample. | | | | |

*21-day Re-inoculation

As can be seen, the antimicrobial product of Example #1 is highly effective against both bacterial and fungal challenges at a concentration of 0.25%. Moreover, the antimicrobial product of Example #1 is not adversely affected by anionics such as Na Lauryl Sulfate.

Product B

INOCULUM

a) Mixed bacteria: Pseud. aeruginosa (ATCC 15442); E.coli (ATCC 8739 or 11229); S. aureus (ATCC 6536).
b) Mixed fungi: A. niger (ATCC 9642); P. luteum (ATCC 9644); C. albicans (ATCC 10231).

| TEST SAMPLE | DAYS | BACTERIA | FUNGI | CONTROL |
|---|---|---|---|---|
| B-1 | 0 | 2,100,000 | 740,000 | <10 |
| | 3 | 2,100,000 | 740,000 | <10 |
| | 7 | 2,100,000 | 740,000 | <10 |
| | 14 | 2,100,000 | 740,000 | <10 |
| | 21* | 2,100,000 | 740,000 | <10 |
| | 28 | 2,100,000 | 740,000 | <10 |
| B-2 | 0 | 1,980,000 | 750,000 | <10 |
| | 3 | 57,000 | 4,200 | <10 |
| | 7 | <10 | 120 | <10 |
| | 14 | <10 | 1,420 | <10 |
| | 21* | <10 | 5,300 | <10 |
| | 28 | <10 | 7,400 | <10 |
| B-3 | 0 | 2,100,000 | 740,000 | <10 |
| | 3 | 12,000 | 3,400 | <10 |
| | 7 | <10 | <10 | <10 |
| | 14 | <10 | <10 | <10 |
| | 21* | <10 | <10 | <10 |
| | 28 | <10 | <10 | <10 |
| B-4 | 0 | 2,100,000 | 700.000 | <10 |
| | 3 | 3,000 | <10 | <10 |
| | 7 | <10 | <10 | <10 |
| | 14 | <10 | <10 | <10 |
| | 21* | <10 | <10 | <10 |
| | 28 | <10 | <10 | <10 |
| NOTE: Control is an uninoculated sample for background count. Bacterial and Fungal Counts are as organisms recovered per gram of sample. Test Day is the number of days after inoculation of the test sample. | | | | |

*21-day Re-inoculation

As can be seen, the antimicrobial product of Example #1 is highly effective against both bacterial and fungal challenges at a concentration of 0.50%. At 0.25%, the product of Example #1 is effective against the bacterial inoculum but failed to completely eradicate the fungi after initial reductions were noted.

Product C

INOCULUM

a) Mixed bacteria: Pseud. aeruginosa (ATCC 15442); E.coli (ATCC 8739 or 11229); S. aureus (ATCC 6536).
b) Mixed fungi: A. niger (ATCC 9642); P. luteum (ATCC 9644); C. albicans (ATCC 10231).

| TEST SAMPLE | DAYS | BACTERIA | FUNGI | CONTROL (Uninoculated) |
|---|---|---|---|---|
| C-1 | 0 | 2,100,000 | 310,000 | 610 |
| | 3 | 2,700,000 | 350,000 | 1,220 |
| | 7 | TNTC* | TNTC | TNTC |
| | 14 | TNTC | TNTC | TNTC |
| | 21 | TNTC | TNTC | TNTC |
| | 28 | TNTC | TNTC | TNTC |
| C-2 | 0 | 2,400,000 | 250,000 | <10 |
| | 3 | <10 | 6,340 | <10 |
| | 7 | <10 | 5,100 | <10 |
| | 14 | <10 | 1,260 | <10 |
| | 21* | <10 | 2,140 | <10 |
| | 28 | <10 | 2,970 | <10 |
| C-3 | 0 | 1,900,000 | 290,000 | <10 |
| | 3 | <10 | 2,170 | <10 |
| | 7 | <10 | <10 | <10 |
| | 14 | <10 | <10 | <10 |
| | 21* | <10 | <10 | <10 |
| | 28 | <10 | <10 | <10 |
| NOTE: Control is an uninoculated sample for background count. Bacterial and Fungal Counts are as organisms recovered per gram of sample. Test Day is the number of days after inoculation of the test sample. | | | | |

* TNTC - Too Numerous to Count
*21-day Re-inoculation

As can be seen, Test sample C-3 (0.5% Product of Example #1) is found to effectively eliminate both bacterial and fungal challenges within seven days of inoculation. The product of Example #1 at 0.5% is capable of functioning effectively as a preservative as measured by the above test parameters.

The antimicrobial test results clearly show the effectiveness of these products in preserving these systems. Noteworthy is the fact that product of Example #1 is not affected by anionics such as sodium lauryl sulfate.

Example 6

Using in vitro test methodology based on the International Planned Parenthood Federation (IPPF) Agreed Test for Total Spermicidal Power as set forth in 21 CFR, Part 351, Volume 45, No. 2/541, December 12, 1980, evidence of spermicidal activity against human sperm is evaluated for contraceptive efficacy.

The product of Example 1 is screened for spermicidal activity by evaluation of 1.0%, 3.0% and 5.0% aqueous solutions thereof.

The 3.0% and 5.0% solutions of the product of Example 1 meet the requirements of the IPPF Agreed test by inactivation of human sperm after ten (10) second contact time.

Example 7

The skin substantivity of the product of Example 1 is evaluated by a multiple wash test protocol.

Individual fingers of selected panelists are washed twice, dried and exposed to the test material. Once exposed, finger imprints are made on agar plates seeded with Staphylococcus epidermidis after which the individual fingers are again washed and dried. A series of four (4) washings and imprints are made, including the initial exposure and imprint. The degree of residual activity or skin substantivity is determined by clarity of inhibition surrounding the imprints on the agar plates (seeded with Staphylococcus epidermidis). A grading system is used to record the data as follows:

0: no activity;
$1^+$: slight activity;
$2^+$: moderate activity;
$3^+$: good;
$4^+$: excellent.

Skin substantivity data are reported in Table III.

TABLE III

| 1.0% Solution Conc. | | | | | | |
|---|---|---|---|---|---|---|
| Panelist | 1 | 2 | 3 | 4 | 5 | Avg. |
| Treated | 4+ | 2+ | 2+ | 3+ | 2+ | 2.6 |
| Wash 1 | 3+ | 2+ | 2+ | 2+ | 1+ | 2.0 |
| Wash 2 | 2+ | 0 | 1+ | 0 | 0 | 0.6 |
| Wash 3 | 1+ | 0 | 0 | 0 | 0 | 0.2 |
| Untreated | NT | 0 | 0 | 0 | 0 | 0.0 |
| 3.0% Solution Conc. | | | | | | |
| Panelist | 1 | 2 | 3 | 4 | 5 | Avg. |
| Treated | 4+ | 4+ | 4+ | 4+ | 4+ | 4.0 |
| Wash 1 | 3+ | 3+ | 3+ | 3+ | 3+ | 3.0 |
| Wash 2 | 3+ | 1+ | 1+ | 2+ | 1+ | 1.6 |
| Wash 3 | 1.5+ | 0 | 0 | 1+ | 0 | 0.5 |
| Untreated | NT | 0 | 0 | 0 | 0 | 0.0 |
| 5.0% Solution Conc. | | | | | | |
| Panelist | 1 | 2 | 3 | 4 | 5 | Avg. |
| Treated | NT | 4+ | 4+ | 4+ | 4+ | 4.0 |
| Wash 1 | NT | 3+ | 4+ | 3+ | 3+ | 3.1 |
| Wash 2 | NT | 3+ | 3+ | 1+ | 2+ | 2.3 |
| Wash 3 | NT | 1+ | 2+ | 0 | 1+ | 1.0 |
| Untreated NT | 0 | | 0 | 0 | 0 | 0.0 |
| NT - not tested | | | | | | |

Example 8

The substantivity of the product of Example 1 to lambskin and latex-type condoms is evaluated by a multiple wash test protocol of the type described in Example 7.

In this study, two (2) cm. squares of prewashed and dried condom materials are exposed to the test materials by dipping into a test solution and blotting to remove excess moisture. Once exposed, the squares are laid on seeded agar plates (seeded with Staphylococcus epidermidis). A series of four (4) washings including the initial exposure are carried out. The degree of residual activity or condom substantivity is determined by the clarity of the zone of

inhibition surrounding the treated and washed squares on the seeded agar plates as compared to the untreated controls. The grading system described in Example 7 is used to record the data obtained.

Lambskin condom substantivity data is reported in Table IV and latex condom substantivity data is reported in Table V.

TABLE IV

| 1.0 % Solution | | | |
|---|---|---|---|
| SWATCH | 1 | 2 | Ave. |
| Treated | 4+ | 4+ | 4.0 |
| Wash 1 | 4+ | 4+ | 4.0 |
| Wash 2 | 4+ | 4+ | 4.0 |
| Wash 3 | 4+ | 4+ | 4.0 |
| Untreated | 0 | 0 | |
| Rating Score | | | 16.0 |
| 3.0 % Solution | | | |
| SWATCH | 1 | 2 | Avg. |
| Treated | 4+ | 4+ | 4.0 |
| Wash 1 | 4+ | 4+ | 4.0 |
| Wash 2 | 4+ | 4+ | 4.0 |
| Wash 3 | 4+ | 4+ | 4.0 |
| Untreated | 0 | 0 | |
| Rating Score | | | 16.0 |
| 5.0 % Solution | | | |
| SWATCH | 1 | 2 | Avg. |
| Treated | 4+ | 4+ | 4.0 |
| Wash 1 | 4+ | 4+ | 4.0 |
| Wash 2 | 4+ | 4+ | 4.0 |
| Wash 3 | 4+ | 4+ | 4.0 |
| Untreated | 0 | 0 | |
| Rating Score | | | 16.0 |

TABLE V

| 1.0 % Solution | | | |
|---|---|---|---|
| SWATCH | 1 | 2 | Avg. |
| Treated | 4+ | 4+ | 4.0 |
| Wash 1 | 2+ | 2+ | 2.0 |
| Wash 2 | 2+ | 1+ | 1.5 |
| Wash 3 | 1+ | 1+ | 1.0 |
| Untreated | 0 | 0 | |
| Rating Score | | | 8.5 |
| 3.0 % Solution | | | |
| SWATCH | 1 | 2 | Avg. |
| Treated | 4+ | 4+ | 4.0 |
| Wash 1 | 3+ | 3+ | 3.0 |
| Wash 2 | 3+ | 2+ | 2.5 |
| Wash 3 | 2+ | 2+ | 2.0 |
| Untreated | 0 | 0 | |
| Rating Score | | | 11.5 |
| 5.0 % Solution | | | |
| SWATCH | 1 | 2 | Avg. |
| Treated | 4+ | 4+ | 4.0 |
| Wash 1 | 4+ | 4+ | 4.0 |
| Wash 2 | 4+ | 4+ | 4.0 |
| Wash 3 | 3+ | 3+ | 3.0 |
| Untreated | 0 | 0 | |
| Rating Score | | | 15.0 |

Example 9

The substantivity of the product of Example 1 to fiber material is evaluated by a multiple wash test protocol of the type described in Example 8 wherein two (2) cm square swatches of fiber material are exposed to the test materials by dipping into the test solution and blotting to remove excess moisture. The exposed samples are layed on seeded agar plates and then subject to the various steps described in Example 8. The grading system described in Example 7 is used to record the data.

The fiber material substantivity data are reported in Table VI.

TABLE VI

| 1.0 % Solution | | | | | |
|---|---|---|---|---|---|
| SWATCH | 1 | 2 | 3 | 4 | Avg. |
| Treated | 4+ | 4+ | 4+ | 4+ | 4.0 |
| Wash 1 | 2+ | 3+ | 2+ | 4+ | 2.8 |
| Wash 2 | 0.5+ | 1+ | 0 | 1+ | 1.0 |
| Wash 3 | 0 | 0 | 0 | 0 | 0.0 |
| Untreated | 0 | 0 | 0 | 0 | 0.0 |
| Rating Score | | | | | 7.8 |
| 3.0 % Solution | | | | | |
| SWATCH | 1 | 2 | 3 | 4 | Avg. |
| Treated | 4+ | 4+ | 4+ | 4+ | 4.0 |
| Wash 1 | 3+ | 2+ | 4+ | 3+ | 3.0. |
| Wash 2 | 1+ | 0 | 1+ | 1+ | 0.8 |
| Wash 3 | 0.5+ | 0 | 0 | 0 | 0.1 |
| Untreated | 0 | 0 | 0 | 0 | 0.0 |
| Rating Score | | | | | 7.9 |
| 5.0 % Solution | | | | | |
| SWATCH | 1 | 2 | 3 | 4 | Avg. |
| Treated | 4+ | 4+ | 4+ | 4+ | 4.0 |
| Wash 1 | 3+ | 4+ | 4+ | 4+ | 3.8 |
| Wash 2 | 1+ | 0.5+ | 0.5+ | 2+ | 1.0 |
| Wash 3 | 1+ | 0 | 0 | 0 | 0.3 |
| Untreated | 0 | 0 | 0 | 0 | 0.0 |
| Rating Score | | | | | 9.1 |

Example 10

The virucidal efficacy of the product of Example 1 against human influenza A virus is demonstrated in this example.

In this test, virucidal efficacy of the test sample is evaluated by reduction in infectivity recoverable from a virus-contaminated surface after exposure to the use-dilution of the product. The test is conducted according to U.S. Environmental Protection Agency guidelines for determining the virucidal efficacy of disinfectants intended for use on dry inanimate surfaces (U.S.E.P.A. Pesticide Assessment Guidelines, Subdivision G: Product Performance, 1982, Section 91-30, pp. 72-76). In order for disinfectant efficacy to be claimed, the following criteria must be met in the test:

1. At least four logs of virus infectivity must be demonstrated, i.e. it must be possible to dilute the virus control four times 10-fold serially and still be able to detect infectious virus in the $10^{-4}$ dilution.
2. The disinfectant must cause a 3 log reduction in virus titer.
3. There can be no detectable virus in the lowest non-toxic dilution of the virus-disinfectant sample.

Human influenza A, strain A2/Hong Kong/8/68, ATCC VR-544, is the virus used in the study of this example. The virus suspension is prepared in allantoic fluid.

The phospholipid compound used in this example is diluted for evaluation on the day of use 1:40 in sterile deionized water.

Fertile chicken eggs incubated at 37 degrees C. are used which are candled on the day of inoculation; only live embryonated eggs being used. The embryonated eggs are inoculated after 10 days of incubation.

The films of virus are made by placing 0.2 ml amounts of undiluted virus suspension on the bottoms of sterile glass Petri dishes and spreading. Films are held at room temperature (approx. 23 degrees C.) and ambient humidity, protected from direct light until dry (approximately 35 minutes).

The dried virus films are treated with 2.0 ml of the use-dilution of the disinfectant sample for an exposure period of 10 minutes at approximately 23 degrees C. After exposure, the bottom of the dish is scraped with a rubber policeman to remove the virus disinfectant mixture.

Concurrently with disinfectant treatment of one virus film, a parallel virus control film is resuspended in 1 ml of Phosphate-buffered saline (PBS).

Assays for virus recovery are carried out by immediately making serial dilutions in PBS with the virus-disinfectant and virus control preparations and subsequently inoculating into embryonated eggs. At least four (4) eggs are used per dilution. The eggs are inoculated with 0.2 ml volumes, and incubated at 37 degrees C. for approximately 72 hours with daily examination for mortality, and then cooled overnight at 4 to 6 degrees C. Allantoic fluids are collected from each egg and centrifuged for 10 minutes at approximately 800 xx g. Hemagglutination (HA) tests are carried out by mixing 0.5 ml of each fluid with 0.5 ml of 0.5% chicken erythrocytes (in PBS) and observing for HA during the next one to two hours at room temperature.

Cytotoxicity controls are run by diluting the use-dilution of the lot of disinfectant sample serially in PBS, and inoculating into embryonated eggs concurrently with virus-disinfectant mixtures. The viability of embryonated eggs is determined daily for three days of incubation at 37 degrees C.

Viral and cytotoxicity titers are reported as - $log_{10}$ of the 50% titration endpoint for infectivity ($ID_{50}$) or toxicity ($TD_{50}$), as calculated by the method of Reed and Miuench (Amer. J. Hyg. 27: 493-497, 1938).

Results of the study are reported in Table XIII.

TABLE XIII

| HUMAN INFLUENZA A VIRUS | | | |
|---|---|---|---|
| Evaluation of the PHOSPHOLIPID Sample for virucidal efficacy against dried virus after a 10-minute exposure to a 1:40 dilution in sterile deionized water. | | | |
| Dilution Inoculated | Control | Hemagglutination (HA) (No. Positive/Inoculated) Sample + Virus | Cytotoxicity Controls (No. Dead/No. Inoculated) |
| $10^{-1}$ | 4/4 | 0/4 | 0/4 |
| $10^{-2}$ | 4/4 | 0/4 | 0/4 |
| $10^{-3}$ | 4/4 | 0/4 | 0/4 |
| $10^{-4}$ | 2/4 | 0/4 | 0/4 |
| Virus Titer (-$log_{10}$) $ID_{50}$) HA Assay | 4/0 | ≤0.5 | |
| Cytotoxicity Titer (-$log_{10}$ $TD_{50}$)- | | | ≥0.5 |
| Reduction of virus titer by test sample (-$log_{10}$ $ID_{50}$)-HA Assay | | ≥3.5 | |

Based on the results of infectivity and cytotoxicity assays shown in Table XIII, the Phospholipid example demonstrates virucidal activity against human influenza A. Infectivity is not detected in the virus-disinfectant mixture at the lowest nontoxic dilution. The reduction in virus titer for the phospholipid product of Example 1 is ≥ 3.5 log.

Example 11

The virucidal efficacy of the product of Example 1 against Herpes Simplex, Type 2 is demonstrated in this example.

The virucidal efficacy assay of this example generally employs the assay method of Example 13 except as noted. The virus employed is Herpes Simplex, type 2, ATCC VR-734 prepared in tissue culture medium. The cell cultures used are prepared from Vero cells obtained from Southern Research Institute with the cultures routinely grown in supplemented minimal essential medium (MEM). The cultures are grown and used as monolayers in disposable tissue

culture labware at 37 degrees C in a humidified atmosphere of 5% $CO_2$ in air. After infection, cultures are held in maintenance medium containing the same ingredients with a 2% fetal calf serum.

The reagents, disinfectant test solution and preparation of virus films are as described in Example 13.

Treatment of Virus Films with Disinfectant: - Dried virus films are treated with 2.0 ml of the use-dilution of the disinfectant sample and allowed to remain in contact for a total exposure period of ten minutes at approximately 23 degrees C. After approximately the first 6.5 minutes of exposure, the bottom of the dish is scraped with a rubber policeman, and an aliquot of the virus-disinfectant mixture is immediately added to a Sephadex column for separation of virus from disinfectant by gel filtration. Concurrently with disinfectant treatment of one virus film, a parallel virus control film is resuspended in 2 ml of Phosphate buffered saline (PBS) and an aliquot is applied to a Sephadex column after 6.5 minutes. Sephadex gel filtration is performed generally by the method of Blackwell and Chen (J.AOAC 53: 1229-1236, 1970). The column filtrates are collected and diluted ten-fold serially for assay of infectivity.

Assays for virus recovery are made using dilutions of each virus-disinfectant and control virus preparation. The dilutions are inoculated into cell cultures, at least four cultures per dilution being used. Cell monolayers are inoculated with 0.05 ml and incubated for one hour at 37 degrees C. After absorption, maintenance medium (0.2 ml) is added and cultures are incubated at 37 degrees C. Cultures are scared for cytopathic effects (CPE) at seven days after inoculation.

Cytotoxicity controls of each batch of disinfectant sample are determined by placing 2.0 ml in the bottom of a sterile Petri dish containing a film of 0.2 ml PBS and after about 6.5 minutes an aliquot is filtered through Sephadex. The column filtrates are collected and diluted ten-fold serially for titration of cytotoxicity.

Calculations of results are carried out as described in Example 10.

The results of infectivity and cytotoxicity assays are reported in Table XIV.

TABLE XIV

| Cytopathic-Cytotoxic Effects (No. Positive/No. Inoculated) | | | |
|---|---|---|---|
| Dilution Cytotoxicity Inoculated | Control | Sample + Virus | Controls |
| $10^{-1}$ | 4/4 | 0/4 | 0/4 |
| $10^{-2}$ | 4/4 | 0/4 | 0/4 |
| $10^{-3}$ | 4/4 | 0/4 | 0/4 |
| $10^{-4}$ | 2/4 | 0/4 | 0/4 |
| Virus Titer ($-\log_{10}$) $ID_{50}$) | 4.0 | ≤0.5 | |
| Cytotoxicity Titer ($-\log_{10}$ $TD_{50}$)- | | ≥0.5 | |
| Reduction of virus titer by test sample ($-\log_{10}$ $ID5_0$)- | | ≥3.5 | |

Example 12

In this example, the virucidal efficacy of the product of Example 1 is evaluated as measured by the reduction in infectivity of Human Immunodeficiency Virus, HTLF-$III_{RF}$ strain of HIV-1 using test protocols as described in Example 10.

Preparation of the starting materials:

The RF Strain of HTLV-III human immunodeficiency virus (HIV) is used in this study. The Virus is produced by cultures of RF virus-infected $H_9$ cells (H9/RF) and is concentrated from supernatant culture fluid by high speed centrifugation by the following procedure: cells are first pelleted from a H9/RF culture by centrifugation at 600 x g for 15 minutes at 4 degrees C. The supernatant culture fluid is transferred to 50 ml centrifuge tubes and centrifuged at 32,500 x g. for 90 minutes at 4 degrees C. The supernatant is decanted and the virus pellet is resuspended in 1/100 the original volume of complete RPMI 1640 medium without fetal bovine serum. Resuspended virus pellets are kept at 4 degrees C. until used to prepare virus films.

The disinfectant used in this example is diluted 1:40 on the day of use in sterile deionized water.

Phosphate-buffered saline (PBS) is that of Dulbecco and Vogt, 1954.

Films of virus are made by spreading 0.2 ml amounts of concentrated virus suspension over 28 $cm^2$ on the bottom of sterile glass Petri dishes. Films are held at room temperature (approx. 23 degrees C.) until visibly dry (approximately 45 minutes) and then incubated at 35-37 degrees C. in a dry oven for an additional 30 minutes to increase the level of dryness.

Method of Determining Virucidal Efficacy of Disinfectant

Treatment of Virus Films with Disinfectant: Dried virus films are treated with 2 ml of the diluted disinfectant and allowed to remain in contact for a total exposure period of 10 minutes at approximately 23 degrees C. After about 6.5 minutes of exposure, the treated virus films are filtered in a Sephadex column as described in Example 7. The column filtrates are diluted 10-fold for assay of infectivity.
Treatment of Virus Control Films: A parallel virus film is resuspended in 2 ml of RPMI 1640 medium without fetal bovine serum and antibiotics. After Sephadex filtration, the column filtrate is diluted 10-fold serially for assay of infectivity.
Cytotoxicity Controls: The cytotoxicity of each batch of disinfectant test sample is prepared by placing 2 ml of the diluted disinfectant test sample in the bottom of a sterile Petri dish containing a film of dried PBS (0.2 ml). After about the first 6.5 minutes, an aliquot is filtered through Sephadex and subsequently diluted 10-fold serially for assay of cytotoxicity.
Infectivity Assay: MT2 cells are indicator cells for infectivity assay. The MT2 cells are treated with polybrene (2 g/ml) for 30 minutes at 37 degrees C., collected by centrifuga-tion and plated in 96-well culture plates at approximately 1 X $10^4$ cells per well in 0.15 ml of medium. Dilutions of each of the test and control groups are inoculated (0.05 ml/well) into four replicate cultures of MT2 cells and the cultures are scored for lytic cytopathic effects (CPE) after eight days of incubation at 37 degrees C. Viral and cyctotoxicity titers are expressed in this example as - $\log_{10}$ of the 50% titration endpoint for infectivity ($ID_{50}$) or toxicity ($TD_{50}$), respectively, as calculated by the method of Reed and Muench.
The results of infectivity and cytotoxicity assays are shown in Table XV.

TABLE XV

| CPE Assay with MT2 Cells (Day 8) Cytopathic-Cytotoxic Effects (No. Positive/No. Inoculated) | | | |
|---|---|---|---|
| Dilution Inoculated | Control | Sample + Virus | Cytotoxicity Controls |
| $10^{-1}$ | | Toxic | 0/4 |
| $10^{-2}$ | 4/4 | 0/4 | 0/4 |
| $10^{-3}$ | 4/4 | 0/4 | 0/4 |
| $10^{-4}$ | 0/4 | 0/4 | 0/4 |
| Virus Titer (-$\log_{10}$) $ID_{50}$) | 5.7 | ≤1.5 | |
| Cytotoxicity Titer (-$\log_{10}$ $TD_{50}$)- | | | ≥0.5 |
| Reduction of virus titer by test sample (-$\log_{10}$ $ID5_0$)- | | ≥4.2 | |

The results of infectivity and cytotoxicity demonstrated that the product of Example 1 possessed virucidal activity against HIV-1 in a CPE assay with MT2 cells.
Having now fully described the invention, it will be apparent to one of ordinary skill in the art that many changes and modifications can be made thereto without departing from the scope of invention as set forth herein.

## Claims

1. Antimicrobial agents which exhibit broad spectrum antibacterial, antifungal, spermicidal and virucidal activity of the formula:

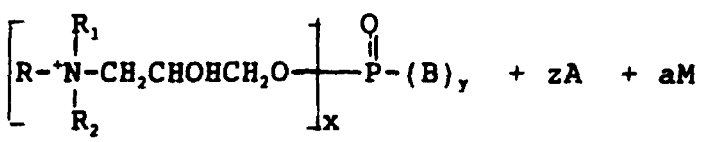

wherein:

x = mixtures of 1 to 3;
x+y = 3
z = x
a = 0 to 2
B = $O^-$ or OM
A = Anion
M is a cation
R, $R_1$ and $R_2$ are the same or different and are alkyl, substituted alkyl, alkyl aryl or alkenyl groups of up to 16 carbon atoms with the proviso that the total carbon atoms in R + $R_1$ + $R_2$ is between 10 and 24.

**2.** Antimicrobial agents as claimed in claim 1, wherein x = mixtures of 2 to 3.

**3.** Antimicrobial agents as claimed in claim 1 or claim 2, wherein $R_1$ and $R_2$ are the same or different alkyl of 1 to 3 carbon atoms.

**4.** Antimicrobial agents as claimed in claim 3, wherein R is alkyl, substituted alkyl or alkenyl groups of 10 to 20 carbon atoms.

**5.** A method of inhibiting the growth of microorganisms which comprises contacting a substrate subject to attack by microorganisms with an antimicrobially effective amount of an antimicrobial compound of the formula:

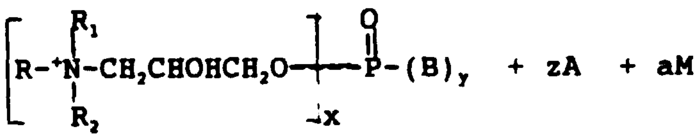

wherein:

x = mixtures of 1 to 3;
x+y = 3 ;
z = x ;
a = 0 to 2;
B = $O^-$ or OM;
A = an anion;
M is a cation;
R, $R_1$ and $R_2$ are the same or different and are alkyl, substituted alkyl, alkyl aryl or alkenyl groups of up to 16 carbon atoms with the proviso that the total carbon atoms in R + $R_1$ + $R_2$ is between 10 and 24.

**6.** Personal care and household cleaning compositions which comprise as one component thereof at least an antimicrobially effective amount of an antimicrobial compound component of the general formula:

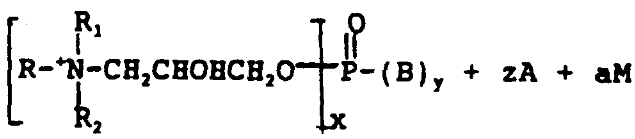

wherein:

x = mixtures of 1 to 3;
x+y = 3 ;
z = x ;
a = 0 to 2;
B = $O^-$ or OM;

A = an anion;
M is a cation;
R, $R_1$ and $R_2$ are the same or different and are alkyl, substituted alkyl, alkyl aryl or alkenyl groups of up to 16 carbon atoms with the proviso that the total carbon atoms in R + $R_1$ + $R_2$ is between 10 and 24.

**7.** Personal care and household cleaning compositions as claimed in claim 6, wherein said antimicrobial compound component is a preservative.

**8.** A method of preparing antimicrobial agents which exhibit broad spectrum antibacterial and antifungal activity of the formula:

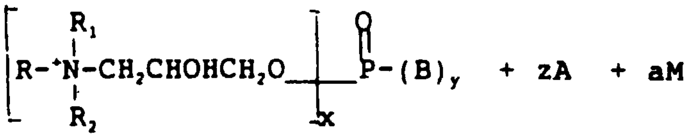

wherein:

x = mixtures of 1 to 3;
x+y = 3 ;
z = x ;
a = 0 to 2;
B = $O^-$ or OM;
A = an anion;
M is a cation;
R, $R_1$ and $R_2$ are the same or different and are alkyl, substituted alkyl, alkyl aryl or alkenyl groups of up to 16 carbon atoms with the proviso that the total carbon atoms in R + $R_1$ + $R_2$ is between 10 and 24.

which comprises:

reacting a phosphate ester reactant with a tertiary amine in the molar ratio of from 1:1 to 3:1 of amine to phosphate ester until the tertiary amine is completely reacted, said phosphate ester reactant being of the general formula:

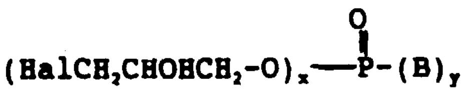

wherein:

x = 1 to 3 or mixtures thereof;
x+y = 3 ;
B = $O^-$ or OM;
Hal = halogen;

and said tertiary amine being of the general formula:

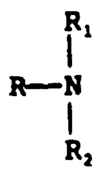

wherein:

R, $R_1$ and $R_2$ are the same or different and are alkyl, substituted alkyl, alkyl aryl or alkenyl groups of up to 16 carbon atoms with the proviso that the total carbon atoms in R + $R_1$ + $R_2$ is between 10 and 24.

**9.** The method as claimed in claim 8, wherein said tertiary amine is reacted with said phosphate ester in the molar ratio of from about 2.0:1 to about 2.5:1 of amine to phosphate ester.

**10.** The method as claimed in claim 8, wherein said tertiary amine is an alkyl dimethylamine wherein the alkyl moiety has from 10 to 20 carbon atoms.

**11.** A method of providing protection to a substrate subject to contact by human and animal sperm and infectious viral organisms which comprises treating a substrate subject to contact by human and animal sperm and infectious viral organisms with an antimicrobially effective mount of an antimicrobial agent selected from a synthetic phospholipid of the formula:

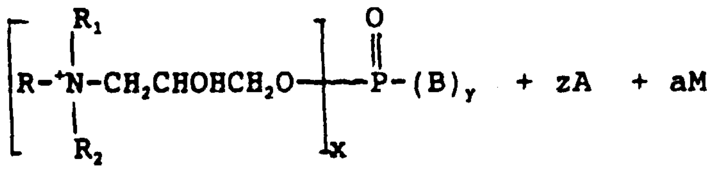

wherein:

x = 1 to 3 or mixtures thereof;
x+y = 3 ;
z = x ;
a = 0 to 2;
B = $O^-$ or OM;
A = an anion;
M is a cation;
R, $R_1$ and $R_2$ are the same or different and are alkyl, substituted alkyl, alkyl aryl or alkenyl groups of up to 16 carbon atoms with the proviso that the total carbon atoms in R + $R_1$ + $R_2$ is between 10 and 24;

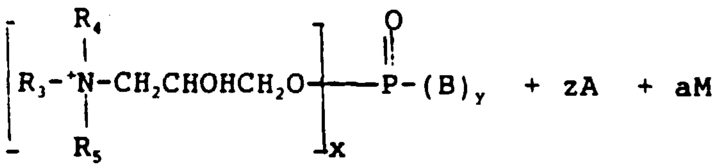

wherein:

x is as hereinabove defined;
x+y = 3 ;
z = x ;
a = 0 to 2;
B = $O^-$ or OM;
A is an anion;
M is a cation;
$R_3$ is an amidoamine moiety of the formula:

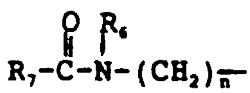

wherein:

$R_7$ is alkyl, alkenyl, alkoxy or hydroxyalkyl of from 5 to 21 carbon atoms each, or aryl or alkaryl of up to 20 carbon atoms;
$R_6$ is hydrogen or alkyl, hydroxyalkyl or alkenyl of up to 6 carbon atoms each or cycloalkyl of up to 6 carbon atoms, preferably of from 2 to 5 carbon atoms, or polyoxyalkylene of up to 10 carbon atoms; and
n is an integer from 2 to 6; and

$R_4$ and $R_5$, which may be the same or different, are selected from alkyl, hydroxyalkyl, carboxyalkyl of up to 6 carbon atoms in each alkyl moiety, and polyoxyalkylene of up to 10 carbon atoms; in addition $R_4$ and $R_5$ taken together with the nitrogen to which they are attached may represent an N-heterocycle;

or mixtures thereof.

**12.** A method of providing spermicidal and virucidal protection to a substrate subject to contact by human and animal sperm and infectious viral organisms which comprises treating a substrate subject to contact by human and animal sperm and infectious viral organisms with an antimicrobially effective amount of a antimicrobial agent comprising a synthetic phospholipid of the formula:

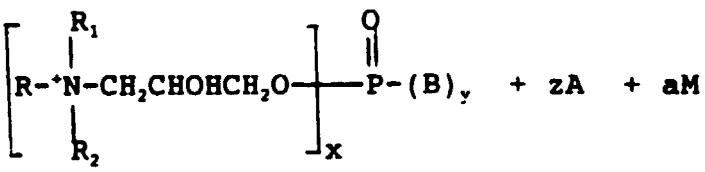

wherein:

x = 1 to 3 or mixtures thereof;
x+y = 3 ;
z = x ;
a = 0 to 2;
B = $O^-$ or OM;
A = an anion;
M is a cation;
R, $R_1$ and $R_2$ are the same or different and are alkyl, substituted alkyl, alkyl aryl or alkenyl groups of up to 16 carbon atoms with the proviso that the total carbon atoms in R + $R_1$ + $R_2$ is between 10 and 24.

**Patentansprüche**

**1.** Antimikrobielle Mittel, welche breites Spektrum von antibakterieller, antifungaler, spermizider und viruzider Aktivität zeigen, der Formel:

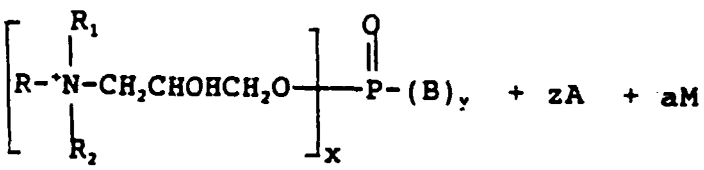

wobei :

x = Mischungen von 1 bis 3;
x+y = 3
z = x

a = 0 bis 2
B = $O^-$ oder OM
A = Anion
M ist ein Kation
R, $R_1$ und $R_2$ sind gleich oder unterschiedlich und sind Alkyl-, substituiertes Alkyl-, Alkylaryl- oder Alkenylgruppen von bis zu 16 Kohlenstoffatomen, unter der Voraussetzung, daß die gesamten Kohlenstoffatome in R + $R_1$ + $R_2$ zwischen 10 und 24 sind.

**2.** Antimikrobielle Mittel nach Anspruch 1, wobei x = Mischungen von 2 bis 3.

**3.** Antimikrobielle Mittel nach Anspruch 1 oder Anspruch 2, wobei $R_1$ und $R_2$ das gleiche oder unterschiedliche Alkyl von 1 bis 3 Kohlenstoffatomen sind.

**4.** Antimikrobielle Mittel nach Anspruch 3, wobei R Alkyl-, substituierte Alkyl- oder Alkenylgruppen von 10 bis 20 Kohlenstoffatomen ist.

**5.** Verfahren zum Inhibieren des Wachstums von Mikroorganismen, welches umfaßt in Kontakt bringen eines Substrats, das Gegenstand durch Angriff von Mikroorganismen ist, mit einer antimikrobiell wirksamen Menge einer antimikrobiellen Verbindung der Formel:

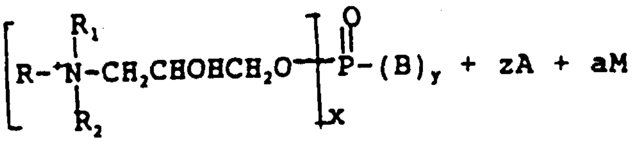

wobei:

x = Mischungen von 1 bis 3;
x+y = 3 ;
z = x ;
a = 0 bis 2;
B = $O^-$ oder OM;
A = ein Anion;
M ist ein Kation;
R, $R_1$ und $R_2$ sind gleich oder unterschiedlich und sind Alkyl-, substituiertes Alkyl-, Alkylaryl- oder Alkenylgruppen mit bis zu 16 Kohlenstoffatomen, unter der Voraussetzung, daß die gesamten Kohlenstoffatome in R + $R_1$ + $R_2$ zwischen 10 und 24 sind.

**6.** Körperpflege- und Haushaltsreinigungszusammensetzungen, welche als eine Komponente davon mindestens eine antimikrobiell wirksame Menge einer antimikrobiellen Verbindungskomponente der allgemeinen Formel umfassen:

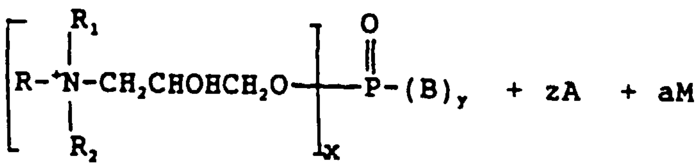

wobei :

x = Mischungen von 1 bis 3;
x + y = 3 ;
z = x ;
a = 0 bis 2; B = $O^-$ oder OM;

A = ein Anion;
M ist ein Kation;
R, $R_1$ und $R_2$ sind gleich oder unterschiedlich und sind Alkyl-, substituiertes Alkyl-, Alkylaryl- oder Alkenylgruppen mit bis zu 16 Kohlenstoffatomen, unter der Voraussetzung, daß die gesamten Kohlenstoffatome in R + $R_1$ + $R_2$ zwischen 10 und 24 sind.

**7.** Körperpflege- und Haushaltsreinigungszusammensetzungen nach Anspruch 6, wobei die antimikrobielle Verbindungskomponente ein Konservierungsnmittel ist.

**8.** Verfahren zum Herstellen von antimikrobiellen Mitteln, welche breites Spektrum von antibakterieller und antifungaler Aktivität zeigen, der Formel

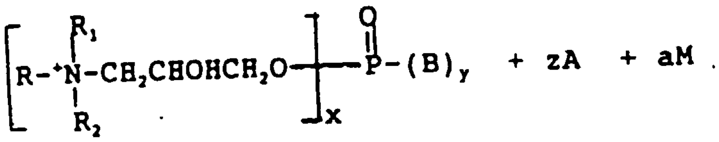

wobei

x = Mischungen von 1 bis 3:
x+y = 3 ;
z = x ;
a = 0 bis 2;
B = $O^-$ oder OM;
A = ein Anion;
M ist ein Kation;
R, $R_1$ und $R_2$ sind gleich oder unterschiedlich und sind Alkyl-, substituiertes Alkyl-, Alkylaryl- oder Alkenylgruppen mit bis zu 16 Kohlenstoffatomen, unter der Voraussetzung, daß die gesamten Kohlenstoffatome in R + $R_1$ + $R_2$ zwischen 10 und 24 sind,

welches umfaßt:

Umsetzen eines Phosphatesterreaktanten mit einem tertiären Amin in dem Molverhältnis von 1:1 bis 3:1 von Amin zu Phosphatester, bis das tertiäre Amin vollständig umgesetzt ist, wobei der Phosphatesterreaktant von der allgemeinen Formel ist:

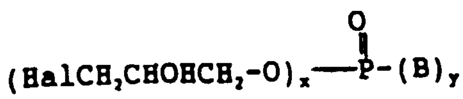

wobei:

x = 1 bis 3 oder Mischungen davon;
x+y = 3 ;
B = $O^-$ oder OM;
Hal = Halogen;

und das tertiäre Amin ist von der allgemeinen Formel:

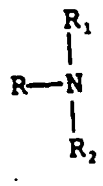

wobei:

R, $R_1$ und $R_2$ gleich oder unterschiedlich sind und Alkyl-, substituiertes Alkyl-, Alkylaryl- oder Alkenylgruppen bis zu 16 Kohlenstoffatomen sind, unter der Voraussetzung, daß die gesamten Kohlenstoffatome in R + $R_1$ +$R_2$ zwischen 10 und 24 sind.

**9.** Verfahren nach Anspruch 8, wobei das tertiäre Amin mit dem Phosphatester in dem Molverhältnis von etwa 2,0:1 bis etwa 2,5:1 von Amin zu Phosphatester umgesetzt wird.

**10.** Verfahren nach Anspruch 8, wobei das tertiäre Amin ein Alkyldimethylamin ist, wobei der Alkylrest 10 bis 20 Kohlenstoffatome hat.

**11.** Verfahren zum zur Verfügung stellen von Schutz einem Substrat, das Gegenstand von Kontakt durch menschliches und tierisches Spermium und infektiöse virale Organismen ist, welches umfaßt Behandeln eines Substrats, das Gegenstand von Kontakt durch menschliches und tierisches Spermium und infektiöse virale Organismen ist, mit einer antimikrobiell wirksamen Menge eines antimikrobiellen Mittels, ausgewählt aus einem synthetischen Phospholipid der Formel:

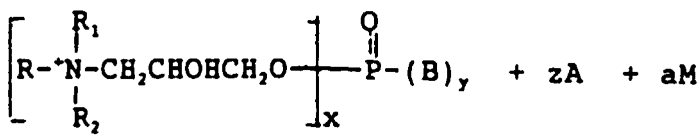

wobei

x = 1 bis 3 oder Mischungen davon;
x+y = 3 ;
z = x ;
a = 0 bis 2;
B = $O^-$ oder OM;
A = ein Anion;
M ist ein Kation,
R, $R_1$ und $R_2$ sind gleich oder unterschiedlich und sind Alkyl-, substituiertes Alkyl-, Alkylaryl- oder Alkenylgruppen mit bis zu 16 Kohlenstoffatomen, unter der Voraussetzung, daß die gesamten Kohlenstoffatome in R + $R_1$ + $R_2$ zwischen 10 und 24 sind;

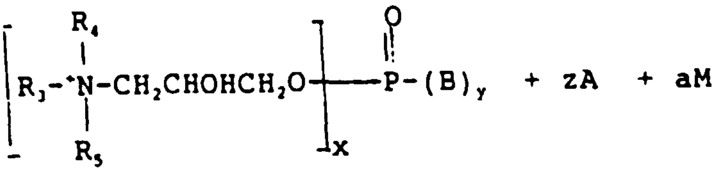

wobei:

x wie hier zuvor angegeben definiert ist;
x+y = 3 ;
z = x ;
a = 0 bis 2;
B = $O^-$ oder OM;
A ist ein Anion;
M ist ein Kation;
$R_3$ ist ein Amidoaminrest der Formel:

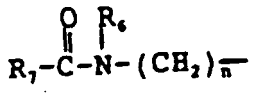

wobei:
$R_7$ Alkyl, Alkenyl, Alkoxy oder Hydroxyalkyl mit 5 bis 21 Kohlenstoffatomen jeweils oder Aryl oder Alkaryl mit bis zu 20 Kohlenstoffatomen ist;
$R_6$ ist Wasserstoff oder Alkyl, Hydroxyalkyl oder Alkenyl mit bis zu 6 Kohlenstoffatomen jeweils oder Cycloalkyl mit bis zu 6 Kohlenstoffatomen, vorzugsweise mit 2 bis 5 Kohlenstoffatomen, oder Polyoxyalkylen mit bis zu 10 Kohlenstofftaomen, und
n ist eine ganze Zahl von 2 bis 6; und $R_4$ und $R_5$, welche gleich oder unterschiedlich sein können, sind ausgewählt aus Alkyl, Hydroxyalkyl, Carboxyalkylvon mit zu 6 Kohlenstoffatomen in jedem Alkylrest, und Polyoxyalkylen mit bis zu 10 Kohlenstoffatomen; zusätzlich können $R_4$ und $R_5$,

zusammengenommen mit dem Stickstoff, an den sie angefügt sind, einen N-Heterozyklus bilden;

oder Mischungen davon.

**12.** Verfahren zum zur Verfügung stellen von spermizidem und viruzidem Schutz einem Substrat, das Gegenstand von Kontakt durch menschliches und tierisches Spermium und infektiöse virale Organismen ist, welches umfaßt, Behandeln eines Substrats, das Gegenstand von Kontakt durch menschliches und tierisches Spermium und infektiöse virale Organismen ist, mit einer antimikrobiell wirksamen Menge eines antimikrobiellen Mittels, umfassend ein synthetisches Phospholipid der Formel:

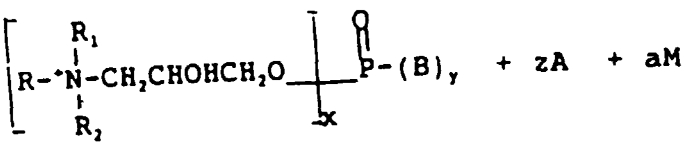

wobei:

x = 1 bis 3 oder Mischungen davon;
x+y = 3 ;
z = x ;
a = 0 bis 2;
B = $O^-$ oder OM;
A = ein Anion;
M ist ein Kation;
R, $R_1$ und $R_2$ sind gleich oder unterschiedlich und sind Alkyl-, substituiertes Alkyl-, Alkylaryl- oder Alkenylgruppen mit bis zu 16 Kohlenstoffatomen, unter der Voraussetzung, daß die gesamten Kohlenstoffatome in R + $R_1$ + $R_2$ zwischen 10 und 24 sind.

**Revendications**

**1.** Agents antimicrobiens qui présentent une activité antibactérienne, fongicide, spermicide et virocide à large spectre

de formule :

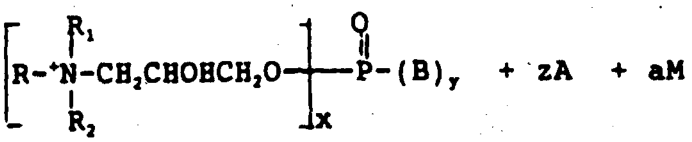

dans laquelle :

x = mélanges de 1 à 3 ;

x+y = 3

z = x

a = 0 à 2

B = $O^-$ ou OM

A = Anion

M est un cation

R, $R_1$ et $R_2$ sont identiques ou différents et sont des groupes alkyles, alkyles substitués, alkylaryles ou alcényles de jusqu'à 16 atomes de carbone à condition que le total des atomes de carbone dans R + $R_1$ + $R_2$ soit compris entre 10 et 24.

**2.** Agents antimicrobiens selon la revendication 1, dans lequels x = mélanges de 2 à 3.

**3.** Agents antimicrobiens selon la revendication 1 ou la revendication 2, dans lesquels $R_1$ et $R_2$ sont un alkyle identique ou différent de 1 à 3 atomes de carbone.

**4.** Agents antimicrobiens selon la revendication 3, dans lesquels R représente des groupes alkyles, alkyles substitués ou alcényles de 10 à 20 atomes de carbone.

**5.** Une méthode d'inhibition de la croissance des micro-organismes qui comprend la mise en contact d'un substrat susceptible d'être attaqué par des micro-organismes avec une quantité antimicrobienne efficace d'un composé antimicrobien de formule :

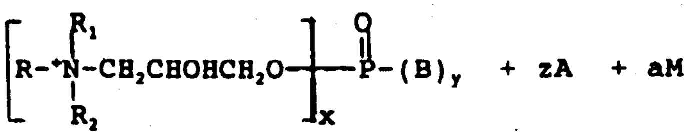

dans laquelle :

X = mélanges de 1 à 3

x+y = 3 ;

z = x ;

a = 0 à 2 ;

B = $O^-$ ou OM ;

A = un anion ;

M est un cation ;

R, $R_1$ et $R_2$ sont identiques ou différents et représentent des groupes alkyles, alkyles substitués, alkylaryles ou alcényles de jusqu'à 16 atomes de carbone à condition que le total des atomes de carbone dans R + $R_1$ + $R_2$ soit compris entre 10 et 24.

**6.** Compositions pour le soin des personnes et de nettoyage ménager qui comprennent sous forme d'un composant de celles-ci au moins une quantité antimicrobienne efficace d'un composant de composé antimicrobien de formule générale :

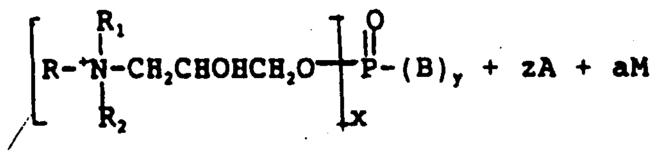

dans laquelle :

X = mélanges de 1 à 3

x+y = 3 ;

z = x ;

a = 0 à 2 ;

B = $O^-$ ou OM ;

A = un anion ;

M est un cation ;

R, $R_1$ et $R_2$ sont identiques ou différents et représentent des groupes alkyles, alkyles substitués, alkylaryles ou alcényles de jusqu'à 16 atomes de carbone à condition que le total des atomes de carbone dans R + $R_1$ + $R_2$ soit compris entre 10 et 24.

**7.** Compositions pour le soin des personnes et de nettoyage ménager selon la revendication 6, dans lesquelles ledit composant du composé antimicrobien est un agent préservatif.

**8.** Une méthode de préparation des agents antimicrobiens qui présentent une activité antibactérienne et fongicide à large spectre de formule :

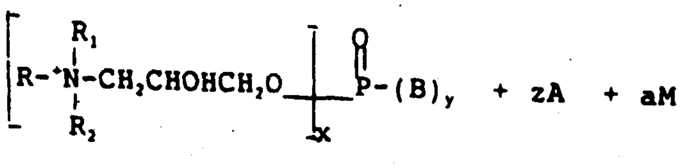

dans laquelle :

X = mélanges de 1 à 3

x+y = 3 ;

z = x ;

a = 0 à 2 ;

B = $O^-$ ou OM ;

A = un anion ;

M est un cation ;

R, $R_1$ et $R_2$ sont identiques ou différents et représentent des groupes alkyles, alkyles substitués, alkylaryles ou alcényles de jusqu'à 16 atomes de carbone à condition que le total des atomes de carbone dans R + $R_1$ + $R_2$ soit compris entre 10 et 24.

qui comprend :

faire réagir un réactif de phosphate ester avec une amine tertiaire en un rapport molaire compris entre 1:1 et 3:1 d'amine par rapport au phosphate ester jusqu'à ce que l'amine tertiaire ait entièrement réagi, ledit réactif de phosphate ester étant de formule générale :

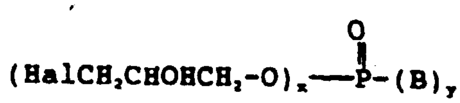

dans laquelle :

X = 1 à 3 ou des mélanges de ceux-ci

x+y = 3 ;

B = $O^-$ ou OM ;

Hal = halogène ;

et ladite amine tertiaire étant de formule générale :

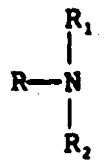

dans laquelle :

R, $R_1$ et $R_2$ sont identiques ou différents et représentent des groupes alkyles, alkyles substitués, alkylaryles ou alcényles jusqu'à 16 atomes de carbone à condition que le total des atomes de carbone dans R + $R_1$ + $R_2$ soit compris entre 10 et 24.

**9.** Méthode selon la revendication 8, dans laquelle ladite amine tertiaire réagit avec ledit phosphate ester en un rapport molaire compris entre environ 2,0:1 et environ 2,5:1 de l'amine par rapport au phosphate ester.

**10.** Méthode selon la revendication 8, dans laquelle ladite amine tertiaire est une alkyldiméthylamine dans lequel le fragment alkyle possède entre 10 et 20 atomes de carbone.

**11.** Méthode pour fournir une protection à un substrat susceptible d'entrer en contact avec du sperme humain et animal et des organismes infectieux viraux qui comprend le traitement d'un substrat susceptible d'entrer en contact par du sperme humain et animal et des organismes infectieux viraux avec une quantité antimicrobienne efficace d'un agent antimicrobien choisi parmi un phospholipide synthétique de formule :

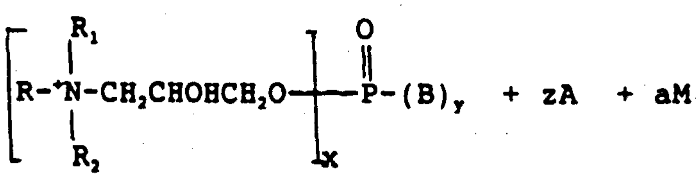

dans laquelle :

X = 1 à 3 ou des mélanges de ceux-ci

$x+y = 3$ ;

$z = x$ ;

a = 0 à 2 ;

B = $O^-$ ou OM ;

A = un anion ;

M est un cation ;

R, $R_1$ et $R_2$ sont identiques ou différents et représentent des groupes alkyles, alkyles substitués, alkylaryles ou alcényles de jusqu'à 16 atomes de carbone à condition que le total des atomes de carbone dans R + $R_1$ + $R_2$ soit compris entre 10 et 24 ;

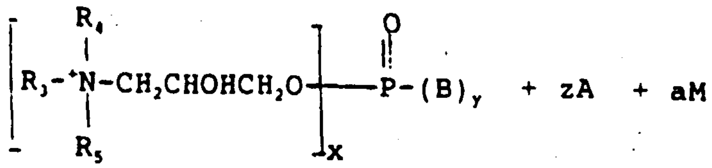

dans laquelle :

X est tel que défini ci-dessus ;

x+y = 3 ;

z = x ;

a = 0 à 2 ;

B = $O^-$ ou OM ;

A est un anion ;

M est un cation ;

$R_3$ est un fragment d'amido-amine de formule :

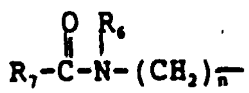

dans laquelle :

$R_7$ représente un alkyle, alcényle, alkoxy ou hydroxyalkyle entre 5 et 21 atomes de carbone chacun, ou un aryle ou alkaryle jusqu'à 20 atomes de carbone ;

R6 représente un hydrogène ou un alkyle, hydroxyalkyle ou alcényle jusqu'à 6 atomes de carbone chacun, ou un cycloalkyle jusqu'à 6 atomes de carbone, de préférence entre 2 et 5 atomes de carbone, ou polyoxyalkylène jusqu'à 10 atomes de carbone; et

n représente un nombre entier entre 2 et 6; et

$R_4$ et $R_5$, qui peuvent être identiques ou différents, sont choisis parmi les alkyles, hydroxyalkyles, carboxyalkyles jusqu'à 6 atomes de carbone dans chaque fragment alkyle, et un polyoxyalkylène jusqu'à 10 atomes de carbone; de plus $R_4$ et $R_5$ pris ensemble avec l'azote auquel ils sont attachés peuvent représenter un N-hétérocycle; ou des mélanges de celui-ci.

**12.** Méthode pour fournir une protection spermicide et virocide à un substrat susceptible d'entrer en contact avec du sperme humain et animal et des organismes infectieux viraux qui comprend le traitement d'un substrat susceptible d'entrer en contact avec du sperme humain et animal et des organismes infectieux viraux avec une quantité antimicrobienne efficace d'un agent antimicrobien comprenant un phospholipide synthétique de formule :

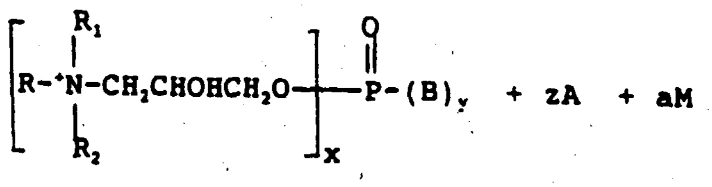

dans laquelle :

X = 1 à 3 ou des mélanges de ceux-ci

x+y = 3 ;

z = x ;

a = 0 à 2 ;

B = $O^-$ ou OM ;

A = un anion ;

M est un cation ;

R, $R_1$ et $R_2$ sont identiques ou différents et représentent des groupes alkyles, alkyles substitués, alkyles aryles ou alcényles jusqu'à 16 atomes de carbone à condition que le total des atomes de carbone dans R + $R_1$ + $R_2$ soit compris entre 10 et 24.